(19) Europäisches Patentamt
European Patent Office
Office européen des brevets

(11) EP 0 545 275 B1

(12) **FASCICULE DE BREVET EUROPEEN**

(45) Date de publication et mention de la délivrance du brevet:
**10.07.1996 Bulletin 1996/28**

(51) Int. Cl.[6]: **C01B 3/38**

(21) Numéro de dépôt: **92120156.2**

(22) Date de dépôt: **26.11.1992**

(54) **Procédé et dispositif pour la fabrication de gaz de synthese et application**

Verfahren und Vorrichtung zur Herstellung von Synthesegas und deren Anwendung

Process and device for manufacturing synthesis gas and their application

(84) Etats contractants désignés:
**BE DE ES GB IT NL**

(30) Priorité: **03.12.1991 FR 9115060**

(43) Date de publication de la demande:
**09.06.1993 Bulletin 1993/23**

(73) Titulaire: **INSTITUT FRANCAIS DU PETROLE**
**F-92502 Rueil-Malmaison (FR)**

(72) Inventeurs:
- **Boucot, Pierre**
  **F-69360 Ternay (FR)**
- **Gateau, Paul**
  **F-44640 Saint Jean de Boisseau (FR)**
- **Weill, Jérôme**
  **F-69005 Lyon (FR)**

(56) Documents cités:
**EP-A- 0 303 438**
**BE-A- 489 995**
**BE-A- 552 748**
**GB-A- 775 334**
**US-A- 2 171 596**

EP 0 545 275 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Rank Xerox (UK) Business Services
2.12.4/3.4

## Description

La présente invention concerne un procédé et un dispositif destiné à la fabrication de gaz de synthèse, utilisable pour produire par exemple : de l'ammoniac, du méthanol, de l'urée, des hydrocarbures, etc..

Les gaz obtenus selon l'invention peuvent être convertis puis éventuellement purifiés ou encore être employés comme gaz réducteurs.

De façon connue, le gaz de synthèse est obtenu par réaction d'un mélange d'hydrocarbures ou combustible avec un oxydant.

Une première façon de produire du gaz de synthèse consiste à associer un réformage primaire avec un réformage secondaire. Le réacteur de reformage primaire est constitué, de façon classique, de tubes remplis de catalyseur et chauffés soit par une combustion externe, soit par échange de chaleur avec des effluents chauds, par exemple avec ceux du réacteur de réformage secondaire. On introduit généralement, dans le réacteur de réformage primaire, l'hydrocarbure avec un fort excès de vapeur.

Les effluents résultant du réformage primaire sont introduits ensuite dans le réformeur secondaire, lequel reçoit également un apport d'oxydant.

Le brevet US-3.278.452 décrit un réformeur secondaire dont l'amélioration consiste en une introduction supplémentaire d'oxydant entre des lits catalytiques disposés successivement dans ce réacteur. L'amélioration apportée par l'étagement de l'oxydant ne résoud cependant pas le principal inconvénient de ce type de réacteurs qui nécessite une importante quantité de vapeur dont la production est coûteuse. L'excès de vapeur a de plus, l'inconvénient de modifier la répartition entre l'hydrogène, le gaz carbonique et l'oxyde de carbone présents dans le gaz de synthèse.

Une autre façon de fabriquer du gaz de synthèse, avec une faible consommation de vapeur, consiste à réaliser une oxydation partielle des hydrocarbures.

Le brevet US-4.699.631 montre un tel exemple de réacteur qui opère sans catalyseur, par voie de flamme. Cependant, ce type de réacteur produit toujours une certaine proportion de suie due à la combustion en défaut d'oxygène, ce qui nécessite ensuite une épuration coûteuse. D'autre part, si l'on souhaite diminuer la quantité de suie, on est obligé d'augmenter la consommation d'oxygène, ce qui réduit le rendement du réacteur. Ainsi, bien qu'opérant avec peu de vapeur, ce type de réacteur présente l'inconvénient de la formation de suies lorsque l'on réduit la consommation d'oxygène ou encore lorsque l'on opère à l'air.

Par ailleurs, la demanderesse a protégé par la demande de brevet EN. 91/09.214 un réacteur du type défini en tête de la description et comportant une chambre dite "à court temps de séjour", c'est-à-dire, telle que $V < 0{,}4D/P$ ; V étant le volume intérieur de la chambre ; D étant le débit massique total entrant dans la chambre de combustion ; P étant la pression régnant à l'intérieur de la chambre. Le but d'un tel réacteur est de réduire l'exigence en vapeur et le coût.

Toutefois, pour des réacteurs dont la chambre de combustion est de trop faible volume, les jets de combustible et de comburant peuvent provoquer une érosion des surfaces vers lesquelles ils sont projetés, après plusieurs milliers d'heures de fonctionnement.

L'objet de l'invention est de proposer un réacteur de gaz de synthèse dont le volume de la chambre de combustion soit suffisant pour éviter ce type d'érosion.

Par ailleurs, afin d'avoir un réacteur peu exigeant en vapeur, il est apparu nécessaire lorsque le volume de la chambre de combustion est relativement élevé, d'introduire successivement du combustible puis du comburant complémentaires dans le lit catalytique situé en aval de la chambre de combustion.

L'invention a pour objet un procédé de conversion de gaz de synthèse consistant à réaliser à l'intérieur d'un unique réacteur :

- une combustion partielle d'un combustible dans une chambre de combustion (1) non catalytique opérant en défaut d'oxydant ; le combustible étant introduit séparément de l'oxydant dans ladite chambre, et
- une introduction d'oxydant complémentaire au niveau d'un lit catalytique (4) situé en aval de ladite chambre de combustion,

  caractérisé en ce qu'il consiste en outre à introduire du combustible au niveau dudit lit catalytique (4) et en amont de l'oxydant complémentaire du combustible, et en ce que le volume intérieur V de ladite chambre (1) est tel que $V > 0{,}4D / P$

  D étant le débit total massique entrant dans la chambre exprimé en Kg/s,

  P étant la pression régnant à l'intérieur de la chambre exprimée en mégapascals,

  V étant exprimé en mètre cube, et

  l'unité contenue dans le chiffre 0,4 est exprimée en $m^2/s$.

Préférentiellement, le combustible introduit dans la chambre de combustion et dans le lit catalytique est essentiellement constitué d'hydrocarbures pouvant être mélangés à des oxydes de carbone et/ou à de l'hydrogène.

Le comburant peut être de l'oxygène pur, ou encore de l'oxygène mélangé à de l'azote, de la vapeur d'eau, du gaz carbonique. Le comburant peut être aussi un mélange d'oxygène et d'un autre gaz inerte.

Préférentiellement, l'apport global de comburant, défini comme étant le nombre de moles d'oxygène contenues dans le comburant injecté dans le réacteur rapporté au nombre de moles de carbone contenues dans le combustible injecté est compris entre 0,3 et 0,65, et le même apport relatif à l'introduction de comburant dans ladite chambre de combustion est compris entre 0,45 et 0,75.

Avantageusement, le rapport hydrogène/hydrocarbures, défini comme étant le rapport molaire exprimé en

nombre de moles d'hydrogène par rapport au nombre de moles de carbone du combustible introduit dans la chambre de combustion, est inférieur à l'unité.

En outre, de la vapeur d'eau peut être introduite avec le comburant et/ou le combustible. L'apport de vapeur d'eau dans le réacteur, défini comme le nombre de moles d'eau rapporté au nombre de moles de carbone, est inférieur à 1,5.

Le combustible peut être préchauffé, avant son entrée dans la chambre de combustion, entre 100 et 850°C, de préférence entre 600 et 700°C. L'oxydant peut être préchauffé à chaque entrée entre 100 et 900°C, de préférence entre 135 et 750°C. La limite supérieure de cette fourchette (750°) peut être abaissée à 600°C, notamment, dans le cas où l'oxydant est de l'oxygène ou essentiellement de l'oxygène pur.

L'invention concerne également l'application du procédé et/ou du dispositif à la production de méthanol, d'ammoniac, d'hydrocarbures, d'urée, d'acide acétique, d'hydrogène ou d'un gaz réducteur.

D'autres avantages et particularités de l'invention apparaîtront plus clairement à la lecture de la description qui va suivre, faite à titre indicatif et nullement limitatif en référence aux figures annexées selon lesquelles :

- la figure 1 est une coupe longitudinale simplifiée d'un réacteur de type vertical selon l'invention,
- la figure 2 est une coupe longitudinale simplifiée d'un réacteur de type transversal selon l'invention,
- la figure 3 est un schéma d'un exemple de réalisation de l'invention.

Les mêmes références seront utilisées pour les éléments communs aux différents modes de réalisation de l'invention.

Ainsi, selon ces figures, le réacteur est essentiellement constitué d'une chambre de combustion 1 pourvue d'au moins deux entrées distinctes, l'une 2 permettant d'introduire le combustible, l'autre 3 injectant le comburant qui est un oxydant.

Les éléments d'injection 2 et 3 permettent non seulement d'introduire le combustible et le comburant dans ladite chambre 1, mais encore d'y stabiliser la combustion.

Une combustion partielle a lieu dans la chambre de combustion 1 et les effluents issus de cette combustion débouchent directement dans la seconde partie 4 du réacteur qui est remplie d'au moins un lit catalytique.

La deuxième partie 4 du réacteur, encore appelée catalyseur ou lit catalytique dans la suite de la description, fait partie du réacteur puisqu'elle présente une surface commune 5 avec la chambre de combustion 1. Cette surface n'est pas nécessairement horizontale.

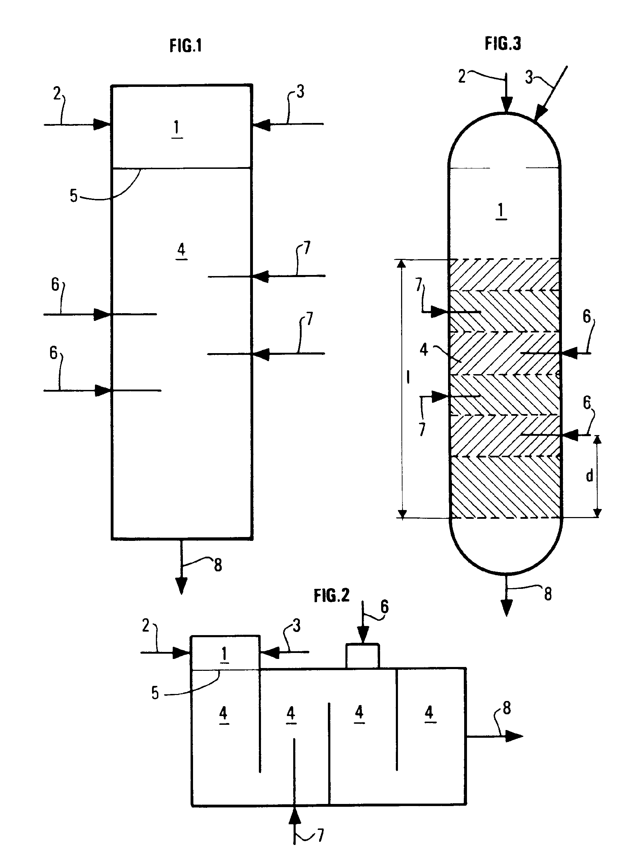

Par ailleurs, le lit catalytique 4 est pourvu d'au moins une entrée 6 pour l'oxydant complémentaire et d'au moins une entrée 7 pour le combustible complémentaire. Sur la figure 1, deux injecteurs 6 et deux injecteurs 7 sont représentés, ce qui constitue un mode de réalisation particulier de l'invention.

Le premier injecteur de combustible 7 se trouve avantageusement positionné en amont du premier injecteur 6 de comburant, relativement au sens de propagation des gaz dans le réacteur.

Enfin, une ou plusieurs sorties 8, placées à l'extrémité du lit catalytique 4 relativement au sens d'écoulement des gaz dans le réacteur, sont bien entendu prévues.

Des injecteurs de tout type connu en soi peuvent être utilisés pour introduire les différents composants qui viennent d'être énoncés.

Les grandes lignes du réacteur selon l'invention étant données, il est nécessaire de préciser certaines conditions de fonctionnement.

La chambre de combustion 1 doit permettre d'opérer à temps de séjour suffisant et en défaut d'oxydant.

Une manière de définir le temps de séjour "suffisant" peut être d'imposer l'inéquation suivante :

$$V > 0,4D / P$$

V étant le volume de la chambre 1 exprimé en mètre cube,

D étant le débit total massique entrant dans la chambre 1 exprimé en Kg/s, et

P étant la pression opératoire prédéterminée régnant dans la chambre 1 exprimée en mégapascals.

Ainsi qu'il est connu de l'homme de métier, et sans que la description qui fait suite puisse être considérée de manière limitative, les catalyseurs utilisés selon la présente invention sont constitués :

- d'un support à base d'oxydes, à propriétés réfractaires et dont l'acidité a été neutralisée,
- d'une phase active comprenant 2 à 40 %, préférentiellement 3 à 30 % en masse d'au moins un métal M réductible choisi parmi le nickel, le cobalt, le chrome, les métaux de la mine du platine. Prise séparément, la proportion des métaux de la mine du platine, s'ils sont présents, varie entre 0,01 et 1 % masse du total ci-avant.

Le support à base d'oxydes comprend au moins un oxyde simple ou mixte de la liste suivante : alumine alpha; aluminate à structure spinelle $NA1_20_4$ - $xA1_20_3$ avec x = 0, 1, 2; d'au moins un métal N choisi dans la liste : magnésium, calcium, strontium, baryum, potassium; aluminate à structure magnétoplombite (ou encore hexaaluminate) $NA1_{12}0_{19}$; N étant un métal de la liste précitée.

Ces supports peuvent en outre, être éventuellement promus par au moins un métal P choisi parmi le silicium, le potassium, l'uranium.

Dans les conditions thermiques les plus sévères, par exemple pour des températures moyennes supérieures à 1000°C, préférentiellement supérieures à 1100°C et très préférentiellement supérieures à 1200°C, il peut se révéler avantageux de disposer en tête une couche d'attaque constituée par exemple d'oxyde de chrome ou

encore d'une faible proportion de nickel déposée sur l'un des supports précités. Ce catalyseur protègera l'autre catalyseur situé dans la couche inférieure comme décrit ci-après.

Les catalyseurs utilisés dans le procédé selon l'invention sont préparés soit par imprégnation du support préformé par une solution contenant au moins un métal M, et éventuellement au moins un métal P, séchage puis activation thermique ; soit encore par malaxage des précurseurs oxydes des métaux aluminium, M et N, éventuellement P, mise en forme, séchage et activation. Le métal P, s'il est présent, peut indifféremment être ajouté avant ou après la mise en forme.

Il est enfin possible de les préparer par coprécipitation ou encore par le procédé sol-gel.

Les catalyseurs utilisés dans le procédé selon l'invention peuvent présenter les géométries les plus variées : pastilles, billes, extrudés, pastilles annulaires, anneaux cannelés, roues de charrette de dimension de 3 à 30 mm. Ils peuvent même être mis en oeuvre sous forme de monolithes constitués, soit par les oxydes et/ou les métaux correspondant aux éléments métalliques précités, soit de monolithes en acier réfractaire revêtus desdits éléments. Un ou plusieurs monolithes peuvent être présents.

Il va de soi que selon les conditions opératoires, la charge utilisée, la composition locale, la présence ou non de vapeur d'eau, le niveau du risque de dépôt de carbone, telle ou telle formule sera utilisée. Ainsi, les catalyseurs promus par le potassium ou encore strontium, ou encore potassium plus calcium, ou encore calcium seront préférentiellement utilisés lorsque le risque de dépôt de carbone est le plus important.

La présente invention est réalisée préférentiellement en présence d'au moins un catalyseur permettant l'activation sélective des processus réactionnels recherchés, soit :

1) convertir sélectivement le méthane et s ils sont également présents, les hydrocarbures supérieurs, par réaction directe ou indirecte avec l'oxygène et/ou la vapeur d'eau présente, en oxydes de carbone et en hydrogène,

2) activer les autres processus réactionnels recherchés et notamment la transformation des précurseurs de coke, selon la réaction

$$C\,H_x + H_2O \rightleftharpoons CO + H_{(2+x)}\; x \geqq 0$$

3) permettre de limiter des réactions de disproportionation du CO

$$2\,CO \rightleftharpoons CO_2 + C$$

par élimination du carbone formé, comme ci-avant,

4) si du CO2 est, au moins pour partie recyclé, activer sélectivement la réaction :

$$CH4 + CO_2 \rightleftharpoons 2CO + 2H2$$

Les catalyseurs connus de l'homme de métier et utilisés indifféremment dans les procédés de vaporéformage, de reformage secondaire, d'oxydation partielle catalytique, conviennent à des titres divers à la réalisation de l'invention. Il est cependant préférable que les catalyseurs utilisés présentent une bonne stabilité thermique (par exemple, jusqu'à au moins 900°C et préférentiellement au moins 1000°C).

En outre, ces catalyseurs peuvent être agencés selon un ou plusieurs lits, disposés comme décrit ci-avant et séparés par un ou plusieurs dispositifs d'injections (6, 7) d'un ou plusieurs composés gazeux tels que décrits ci-dessus.

La vitesse volumétrique horaire (VVH) rapportée à l'hydrocarbure et exprimée en volumes TPN d'hydrocarbure par heure et par volume de catalyseur peut être exprimée en VVH corrigée. Si m est le nombre moyen d'atomes de carbone de la charge, la VVH corrigée (qui sera celle employée dans le procédé de l'invention) s'écrit :

$$\text{VVH corrigée} = \text{VVH} \times m$$

On opère avec une VVH corrigée comprise entre 200 et 10.000 heures$^{-1}$, préférentiellement entre 400 et 8000, et très préférentiellement 500 et 7000 heures$^{-1}$.

Il est évident pour l'homme de métier que le lit de catalyseur peut être séparé en n lits de volumes $V_1$, $V_2$,... $V_i$... $V_n$, tels que $V_1+V_2+...+V_i+...+V_n$ = v, la VVH restant exprimée par rapport au volume total de catalyseur, v.

Le combustible introduit par la ou les entrées 2 de la chambre de combustion et par les entrées 7, sera de préférence constitué d'hydrocarbures (gaz naturel ou méthane par exemple) mélangé à des oxydes de carbone (CO, $CO_2$) et/ou à de l'hydrogène et/ou à des gaz inertes.

De la vapeur d'eau peut en outre être mélangée aux hydrocarbures, de préférence dans la proportion définie en tête de la description.

La proportion d'hydrogène dans les hydrocarbures est telle que le rapport $H_2$/hydrocarbures est inférieur à l'unité.

La composition des gaz injectés aux différentes entrées n'est pas forcément identique.

L'oxydant introduit au niveau de l'entrée 3 peut être de l'oxygène pur, un mélange d'oxygène et d'azote, de l'air, un mélange d'oxygène et de vapeur d'eau, un mélange d'oxygène et de gaz carbonique, un mélange d'oxygène et d'un autre gaz inerte.

L'apport total de vapeur d'eau et de gaz carbonique reste faible vis-à-vis de certaines autres technologies de l'art antérieur, citées plus haut. En effet, on utilisera préférentiellement un rapport molaire

$$K = \frac{H_2O + CO_2}{C} < 1{,}5$$

où C représente tout le carbone compris dans les hydrocarbures, et où ($H_2O$ + $CO_2$) représente la somme des

débits molaires d'eau et de $CO_2$ injectés. A titre de comparaison, le même rapport molaire pris sur un réacteur autotherme classique serait supérieur à deux.

Le fait d'opérer, selon l'invention, avec plusieurs entrées d'oxydant permet de moduler la composition du combustible et de l'oxydant aux différents étages et donc de mieux maîtriser la réaction. Par exemple, pour la synthèse de l'ammoniac, si l'on souhaite opérer avec la stoechiométrie $N_2$ + 3 $H_2$, on introduira au niveau du lit catalytique de l'air par la ou les autres entrées 6.

Un préchauffage est conseillé, à la fois pour le combustible et pour l'oxydant avant leur introduction dans le réacteur. De préférence, le combustible peut être préchauffé entre 100°C et 850°C, tandis que l'oxydant peut subir un préchauffage compris entre 100 et 900°C. Plus précisément, des températures comprises entre 200°C et 750°C sont à préférer.

La pression dans la chambre de combustion 1 est comprise entre 1 et 150 bars, de préférence entre 30 et 100 bars.

L'intérêt de la présente invention sera mieux compris par la comparaison des exemples qui vont suivre. L'exemple 1 présente des résultats de l'art antérieur tandis que les exemples 2 et 3 illustrent des modes de réalisation de l'invention. Dans tous les exemples qui suivent, le réacteur reçoit du gaz naturel contenant (en volume) 98,7 % de méthane, 0,9 % d'éthane et 0,4 % d'azote.

<u>EXEMPLE 1</u>

Il concerne un réacteur pilote dont le volume total intérieur est de 250 titres (ensemble chambre et catalyseur). Ce réacteur est rempli à moitié de catalyseur pour laisser à la chambre un volume de 125 litres.

Le lit catalytique comporte en tête une première couche d'un catalyseur contenant 3,8 % de chrome sur alumine alpha. Cette couche occupe 20 % du volume total de catalyseur. Le reste est constitué par un catalyseur contenant 8,8 % de nickel également déposé sur alumine alpha.

La chambre de combustion est alimentée par du gaz naturel et de l'oxygène, tous les deux mélangés à de la vapeur et introduits à 777 K. Le gaz naturel contient 50 % de son débit en vapeur : le débit total (vapeur plus gaz naturel) est de l'ordre de 150 $Nm^3/h$. L'oxygène pur dont le débit est de 58 $Nm^3/h$ est mélangé à de la vapeur dont le débit est de 195 $Nm^3/h$.

La pression dans le réacteur est de 30 bars.

La température sur la première couche de catalyseur est de 1453 K.

Le débit de gaz naturel a pu être porté de 100 à 112 $Nm^3/h$ (avec 50 $Nm^3$ /h de vapeur) et le débit de vapeur introduit avec l'oxygène a pu être abaissé de 195 $Nm^3/h$ à 170 $Nm^3/h$.

La température en tête de lit atteint alors 1476 K.

La composition de sortie est la suivante :

| | |
|---|---|
| $H_2$ | 42,8 % |
| $CO_2$ | 7,2 % |
| $CH_4$ | 0,6 % |
| CO | 12,4 % |
| $H_2O$ | 37 % |

Avec un tel réacteur, on ne peut pas réduire le débit de vapeur au-dessous de 160 $Nm^3/h$, coté oxygène, sans provoquer une augmentation de la perte de charge due à un chargement du catalyseur en suie.

<u>EXEMPLE 2</u>

L'exemple ci-dessus, selon l'art antérieur, montre qu'il n'est pas possible d'atteindre un rapport $H_2/CO$ voisin de 2, ce qui est une condition nécessaire pour la fabrication d'hydrocarbures supérieurs par des procédés de type Fisher-Tropsch.

Le réacteur selon ce second exemple est identique à celui de l'exemple 1, ainsi que les débits arrivant à la chambre de combustion 1. Par ailleurs, les volumes de la chambre 1 et du catalyseur 4 restent inchangés.

Toutefois, dans le lit catalytique 4, au deux-tiers de la hauteur à partir de la sortie 8, débouchent quatre tubes 7 percés d'orifices. Ces tubes sont protégés par une double enveloppe refroidie à la vapeur. Dans cette partie du lit catalytique, la température est de 1253 K.

Par les tubes 7, arrive un mélange de 112 $Nm^3/h$ de gaz naturel et 22 $Nm^3/h$ de vapeur à 780 K. Le refroidissement des tubes grâce à la vapeur évite un cokage dans les tubes.

A la moitié de la hauteur du lit 4 débouchent en outre quatre tubes 6 percés d'orifices. A la différence des tubes 7 d'alimentation en gaz naturel, les tubes 6 sont en alumine et ne sont pas refroidis.

Le lit catalytique à ce niveau est constitué d'une couche de catalyseur à 3,8 % de chrome.

Par les tubes 6, arrive un mélange d'oxygène, de vapeur et de gaz carbonique tous préchauffés à 765 K.

| | |
|---|---|
| Débit $0_2$ | 65 $Nm^3/h$ |
| Débit vapeur | 24 $Nm^3/h$ |
| Débit $CO_2$ | 62 $Nm^3/h$ |

En sortie de réacteur, la température est de l'ordre de 1245 K., la composition des gaz est la suivante :

| | |
|---|---|
| $H_2$ | 41,9 % |
| $CO_2$ | 8,8 % |
| $CH_4$ | 0,8 % |
| CO | 19,4 % |
| $H_2O$ | 29,1 % |

EXEMPLE 3

Le réacteur selon l'exemple 2 peut être modifié afin de réduire davantage le taux de vapeur nécessaire.

Le réacteur selon l'exemple 3 est un mode de réalisation de l'invention présentant cette caractéristique. La figure 3 illustre ce réacteur.

Ainsi, le volume total du réacteur est de 250 l (0,25 $m^3$). La chambre de combustion 1 présente un volume de 80 litres.

Au niveau de la chambre de combustion 1, par l'entrée 2 destinée au gaz naturel, est introduit du gaz à un débit de 75 $Nm^3/h$ et de la vapeur à un débit de 135 $Nm^3/h$. La température du mélange introduit est d'environ 773 K. Par l'entrée 2 destinée à l'oxydant, est introduit un mélange d'oxygène avec un débit de 45 $Nm^3/h$ et de vapeur ayant un débit de 135$Nm^3/h$, le mélange étant porté à la température moyenne de 793 K.

Dans le catalyseur 4, quatre niveaux d'introduction sont prévus :

- Au niveau le plus proche de la chambre de combustion 1 est introduit un mélange de gaz naturel (débit d'environ 85 $Nm^3/h$) et de vapeur (débit d'environ 17 $Nm^3/h$), à une température proche de 773 K. Quatre tubes 7 peuvent être prévus, à 90° les uns des autres, pour injecter ce mélange.
- Quatre autres tubes débouchent à un deuxième niveau du lit catalytique, tous situés à une certaine même distance du premier niveau. Ces tubes 6 permettent d'introduire un mélange d'oxygène et de vapeur à environ 673 K. Le débit d'oxygène est préférentiellement de 47 $Nm^3/h$, tandis que le débit de vapeur est proche de 25 $Nm^3/h$.

De préférence, les tubes 6 placés à ce second niveau sont angulairement équidistants et ils sont de plus angulairement décalés vis-à-vis des tubes 7 du premier niveau.

Au troisième niveau du lit catalytique, débouchent en outre plusieurs (quatre de préférence) tubes 7 destinés à introduire un mélange gaz naturel-vapeur. Le débit du gaz naturel est de l'ordre de 95 $Nm^3/h$, tandis que le débit vapeur avoisine 19 $Nm^3/h$. Le mélange est introduit à environ 773 K.

Enfin, le quatrième niveau est plus spécifiquement réservé à une introduction d'oxygène pur, à environ 573 K avec un débit de 55 $Nm^3/h$. De préférence, quatre tubes sont alors prévus présentant les mêmes caractéristiques que les tubes des autres niveaux, a savoir angulairement équidistants et angulairement décalés vis-à-vis des tubes du niveau 3.

De préférence, les différents niveaux sont équidistants, placés chacun à une distance, mesurée sur l'axe longitudinal du réacteur, égale au sixième de la hauteur totale du lit catalytique 4.

Le lit catalytique 4 est formé d'une alternance de couches constituées respectivement de 3,8 % de chrome sur alumine alpha et de 8,8 % de nickel sur alumine alpha, comme illustré par la figure 3. Les tubes d'injection 7 situés aux premier et troisième niveaux du lit catalytique débouchent préférentiellement sur le catalyseur contenant 8,8 % de nickel tandis que les tubes 6 situés aux deuxième et quatrième niveaux débouchent dans le catalyseur à 3,8 % de chrome.

De façon préférentielle, la distance d mesurée sur l'axe longitudinal du réacteur, entre le quatrième niveau et l'extrémité du lit 4 coté sortie 8, est de l'ordre du tiers de la hauteur totale du lit.

En sortie du réacteur, selon cet exemple, les gaz sont à une température d'environ 1351 K, avec la composition suivante :

| | |
|---|---|
| $H_2$ | 53,6 % |
| $H_2O$ | 18,5 % |
| $CH_4$ | 0,6 % |
| CO | 23,2 % |
| $CO_2$ | 4,1 % |

Dans cet exemple, on notera que la teneur en gaz carbonique est supérieure aux préconisations d'une synthèse dite de Fisher-Tropsch. Une décarbonatation permettra d'abaisser cette teneur. Le gaz carbonique séparé peut avantageusement être introduit en lieu et place d'une partie de la vapeur d'eau.

**Revendications**

1. Procédé de conversion de gaz de synthèse consistant à réaliser à l'intérieur d'un unique réacteur :

    - une combustion partielle d'un combustible dans une chambre de combustion (1) non catalytique opérant en défaut d'oxydant ; le combustible étant introduit séparément de l'oxydant dans ladite chambre, et
    - une introduction d'oxydant complémentaire au niveau d'un lit catalytique (4) situé en aval de ladite chambre de combustion,

    caractérisé en ce qu'il consiste en outre à introduire du combustible au niveau dudit lit catalytique (4) et en amont de l'oxydant complémentaire du combustible, et en ce que

le volume intérieur V de ladite chambre (1) est tel que V > 0,4D / P

D étant le débit total massique entrant dans la chambre exprimé en Kg/s,

P étant la pression régnant à l'intérieur de la chambre exprimée en mégapascals,

V étant exprimé en mètre cube, et

l'unité contenue dans le chiffre 0,4 est exprimée en $m^2/s$.

2. Procédé selon la revendication 1, caractérisé en ce que l'apport de vapeur d'eau dans le réacteur, défini comme le nombre de moles d'eau rapporté au nombre de moles de carbone, est inférieur à 1,5.

3. Procédé selon l'une quelconque des revendications 1 ou 2, caractérisé en ce que l'on introduit dans la chambre de combustion (1) et dans le lit catalytique (4) un combustible essentiellement constitué d'hydrocarbures pouvant être mélangés à des oxydes de carbone et/ou à de l'hydrogène.

4. Procédé selon la revendication 3, caractérisé en ce que le rapport hydrogène/hydrocarbures, défini comme étant le rapport molaire exprimé en nombre de moles d'hydrogène par rapport au nombre de moles de carbone du combustible introduit dans la chambre de combustion, est inférieur à l'unité.

5. Procédé selon l'une quelconque des revendications précédentes, caractérisé en ce que le comburant est choisi parmi l'oxygène pur, un mélange d'oxygène et d'azote, un mélange d'oxygène et de gaz carbonique, ou un mélange d'oxygène et autres gaz inertes.

6. Procédé selon l'une quelconque des revendications précédentes, caractérisé en ce que l'on introduit par les injecteurs (2, 3, 6, 7) destinés au comburant et au combustible introduisent en outre de la vapeur d'eau.

7. Procédé selon l'une quelconque des revendications précédentes, caractérisé en ce que le combustible est préchauffé, avant son entrée dans la chambre de combustion, entre 100 et 850°C, et en ce que l'oxydant est préchauffé à chaque entrée entre 100 et 900°C

8. Procédé selon l'une quelconque des revendications précédentes, caractérisé en ce que l'apport global de comburant, défini comme étant le nombre de moles d'oxygène contenu dans le comburant injecté dans le réacteur rapporté au nombre de moles de carbone contenues dans le combustible injecté est compris entre 0,3 et 0,65, et en ce que le même apport relatif à l'introduction de comburant dans ladite chambre de combustion est compris entre 0,45 et 0,75.

9. Application du procédé selon l'une des revendications précédentes, à toute fabrication chimique mettant en oeuvre du gaz de synthèse.

10. Application selon la revendication 9, pour la fabrication de l'hydrogène.

11. Application selon la revendication 9, pour la fabrication d'au moins un hydrocarbure supérieur.

12. Application selon la revendication 9, pour la fabrication du méthanol.

**Claims**

1. Method for manufacturing synthetic gas consisting of bringing about within a single reactor :

- a partial combustion of a fuel in a non-catalytic combustion chamber (1) operating without any oxidant; the fuel is introduced into the said chamber separately from the oxidant, and
- additional oxidant is introduced at the level of a catalytic bed (4) located downstream of the said combustion chamber,

characterised in that it consists in addition in introducing fuel at the said level of the catalytic bed (4) and fuel upstream of the additional oxidant and in that the interior volume V of the said chamber (1) is such that V > 0.4D / P

where D is the total mass flow entering the chamber expressed in Kg/s,

P is the pressure prevailing inside the chamber expressed in megapascals,

V is expressed in cubic metres, and

the unit contained in the figure 0.4 is expressed in $m^2/s$.

2. Method as claimed in claim 1, characterised in that the amount of steam fed into the reactor, defined as being the number of moles of water relative to the number of moles of carbon is less than 1.5.

3. Method as claimed in any one of the previous claims 1 or 2, characterised in that a fuel mainly made up of hydrocarbons and possibly mixed with carbon oxides and/or hydrogen is introduced into the combustion chamber (1) and into the catalytic bed (4).

4. Method as claimed in claim 3, characterised in that the hydrogen/hydrocarbon ratio, defined as being the molar ratio expressed by the number of moles of hydrogen relative to the number of moles of carbon in the fuel introduced into the combustion chamber is less than one.

5. Method as claimed in any one of the previous claims, characterized in that the oxidant is selected from among pure oxygen, a mixture of oxygen and nitrogen, a mixture of oxygen and carbon dioxide or a mixture of oxygen and other inert gases.

6. Method as claimed in any one of the previous claims, characterized in that steam is also injected through the injectors (2, 3, 6, 7) intended for the oxidant and fuel.

7. Method as claimed in any one of the previous claims, characterized in that the fuel is pre-heated to between 100 and 850°C before admission to the combustion chamber and in that the oxidant is pre-heated at each intake to between 100 and 900°C.

8. Method as claimed in any one of the previous claims, characterized in that the overall amount of oxidiser, defined as being the number of moles of oxygen contained in the oxidiser injected into the reactor relative to the number of moles of carbon contained in the injected fuel is within the range between 0.3 and 0.65 and in that the same amount with respect to the oxidiser introduced into the combustion chamber is within the range of 0.45 and 0.75.

9. Application of the method as claimed in one of the previous claims, to any chemical production process using synthetic gas.

10. Application as claimed in claim 9 for the manufacture of hydrogen.

11. Application as claimed in claim 9, for the manufacture of at least one high hydrocarbon.

12. Application as claimed in claim 9 for the manufacture of methanol.

**Patentansprüche**

1. Verfahren zur Umwandlung von Synthesegas, das darin besteht, im Inneren eines Einzelreaktors zu bewirken:

- eine teilweise Verbrennung eines Brennmateriales in einer nicht-katalytischen Verbrennungskammer (1), die unter Oxidationsmittelmangel arbeitet, wobei das Brennmaterial getrennt vom Oxidationsmittel in die Kammer eingeführt wird und

- ein Einführen von ergänzendem Oxidationsmittel in ein katalytisches Bett (4), das stromabwärts von der Verbrennungskammer gelegen ist, dadurch gekennzeichnet, daß es des weiteren darin besteht, Brennmaterial in das katalytische Bett (4) und stromaufwärts vom ergänzenden Oxidationsmittel des Brennmaterials einzuführen und dadurch, daß das Innenvolumen V der Kammer (1) gleich $V > 0,4\ D/P$ ist, wobei,

D der in die Kammer eintretende Gesamtmaßendurchsatz, aus gedrückt in kg/s, ist,

P der im Inneren der Kammer herrschende Druck, ausgedrückt in Megapascal, ist,

V in Kubikmeter ausgedrückt ist und

die in der Ziffer 0,4 enthaltene Einheit in $m^2/s$ ausgedrückt ist.

2. Verfahren nach Anspruch 1, dadurch gekennzeichnet, daß das Angebot an Wasserdampf im Reaktor, das als die Zahl von Molen Wasser bezogen auf die Zahl von Molen Kohlenstoff definiert ist, unter 1,5 liegt.

3. Verfahren nach irgendeinem der Ansprüche 1 oder 2, dadurch gekennzeichnet, daß man in die Verbrennungskammer (1) und in das katalytische Bett (4) ein Brennmaterial einführt, das im wesentlichen aus Kohlenwasserstoffen besteht, welche mit Oxiden von Kohlenstoff und/oder mit Wasserstoff vermischt sein können.

4. Verfahren nach Anspruch 3, dadurch gekennzeichnet, daß das Verhältnis Wasserstoff/Kohlenwasserstoffe, das als das in Zahl von Molen Wasserstoff bezogen auf die Zahl von Molen Kohlenstoff des in die Verbrennungskammer eingeführten Brennmaterials ausgedrückte Molverhältnis definiert ist, kleiner als die Einheit ist.

5. Verfahren nach irgendeinem der vorhergehenden Ansprüche, dadurch gekennzeichnet, daß der Sauerstoffträger aus reinem Sauerstoff, einer Mischung aus Sauerstoff und Stickstoff, einer Mischung aus Sauerstoff und kohlensaurem Gas oder einer Mischung aus Sauerstoff und anderen inerten Gasen ausgewählt ist.

6. Verfahren nach irgendeinem der vorhergehenden Ansprüche, dadurch gekennzeichnet, daß man durch die Injektoren (2, 3, 6, 7), welche für den Sauerstoffträger und das Brennmaterial bestimmt sind, des weiteren Wasserdampf einführt.

7. Verfahren nach irgendeinem der vorhergehenden Ansprüche, dadurch gekennzeichnet, daß das Brennmaterial vor seinem Eintritt in die Verbrennungskammer zwischen 100 und 850°C vorerhitzt wird, und dadurch, daß das Oxidationsmittel bei jedem Eingang zwischen 100 und 900°C vorerhitzt wird.

8. Verfahren nach irgendeinem der vorhergehenden Ansprüche, dadurch gekennzeichnet, daß das Gesamtangebot von Sauerstoffträger, das als die Zahl von Molen Sauerstoff, welche in dem in den

Reaktor eingeführten Sauerstoffträger enthalten ist, bezogen zur Zahl von Molen von Kohlenstoff, welche im eingeführten Brennmaterial enthalten ist, zwischen einschließlich 0,3 und 0,65 enthalten ist und dadurch, daß das gleiche Angebot, bezogen auf die Einführung von Sauerstoffträger in die Verbrennungskammer, zwischen einschließlich 0,45 und 0,75 enthalten ist.

**9.** Verwendung des Verfahrens nach einem der vorhergehenden Ansprüche, auf alle chemischen Herstellungen, welche Synthesegas einsetzen.

**10.** Verfahren nach Anspruch 9, zur Herstellung von Wasserstoff.

**11.** Verwendung nach Anspruch 9, zur Herstellung wenigstens eines höheren Kohlenwasserstoffes.

**12.** Verfahren nach Anspruch 9, zur Herstellung von Methanol.

FIG.1

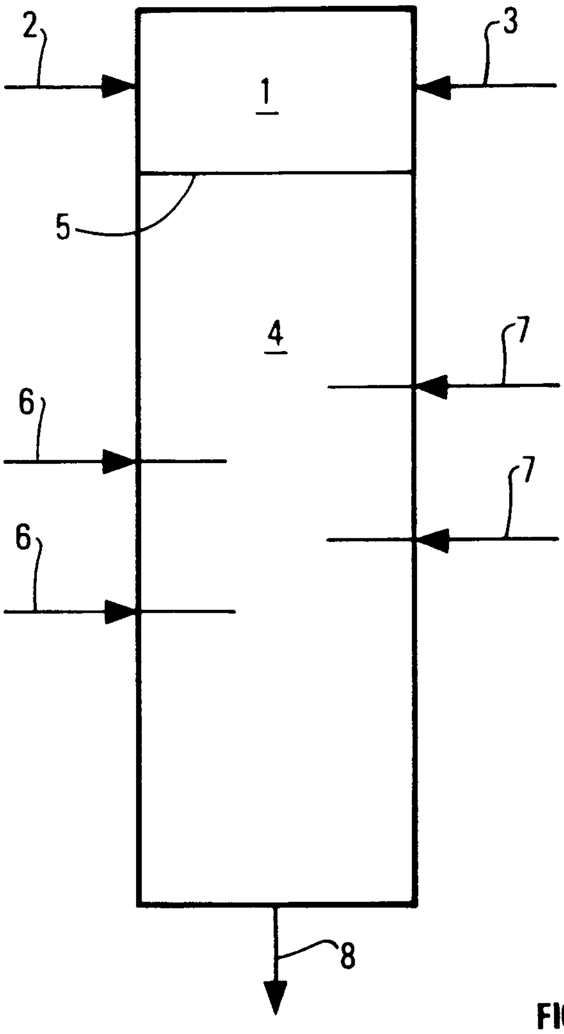


FIG.3

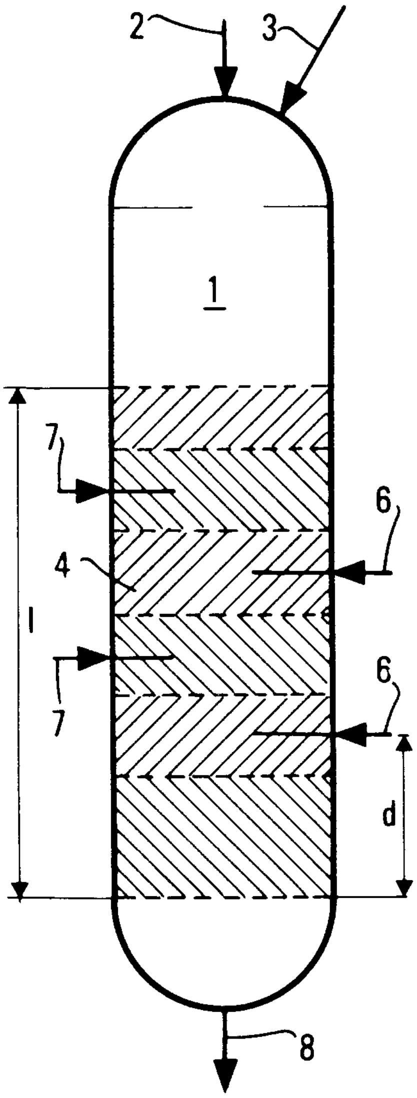


FIG.2